# EUROPÄISCHE PATENTSCHRIFT

(11) **EP 0 848 963 B1**
(45) Veröffentlichungstag und Bekanntmachung des Hinweises auf die Patenterteilung: **27.03.2002**
(21) Anmeldenummer: 97121120.6
(22) Anmeldetag: 02.12.1997
(51) Int. Cl.: A61M 16/08

(54) **Gaszuführleitung**
Gas feed conduit
Conduit d'admission de gaz

(30) Priorität: 05.12.1996 DE 29621157 U
(43) Veröffentlichungstag der Anmeldung: 24.06.1998
(73) Patentinhaber: GOTTLIEB WEINMANN GERÄTE FÜR MEDIZIN UND ARBEITSSCHUTZ GMBH & CO., 22525 Hamburg (DE)
(72) Erfinder: Mandel, Detlef, 22529 Hamburg (DE)
(74) Vertreter: Liebelt, Rolf, Dipl.-Ing.

(56) Entgegenhaltungen:
- EP-A- 0 697 225
- US-A- 4 558 708
- US-A- 4 985 055

## Beschreibung

Die Erfindung betrifft eine Gaszuführleitung, die an eine Atemmaske zur künstlichen Beatmung eines Patienten anschließbar ist und einen Abschnitt aus einem porösen oder feinporigem Werkstoff aufweist, über den Gas an die Umgebung abgegeben wird.

Bei der künstlichen Beatmung eines Patienten ist ein Beatmungsgerät über die Gaszuführleitung, die aus der EP-A-O 697 225 bekannt ist, mit einer Atemmaske verbunden. Die Wandung eines Abschnittes dieser Leitung, über den die Überschußluft und die vom Patienten ausgeatmeten Gase an die Umgebung abgegeben werden, besteht aus einem porösen oder feinporigen Werkstoff. Dieser Abschnitt ist möglichst nahe zur Atemmaske in der Gaszuführleitung vorgesehen, damit die gesamte Menge der ausgeatmeten Gase nicht bis in das Beatmungsgerät zurückströmt. Das Abführen der ausgeatmeten Atemgase über einen porös oder feinporig ausgebildeten Abschnitt der Gaszuführleitung ist besonders bei häufiger und lang andauernder künstlicher Beatmung, z. B. bei der Behandlung einer Schlafapnoe mit Problemen verbunden. In den engen Kanälen mit einem Querschnitt von etwa 50 µm bis 200 µm der Leitung, über welche die ausgeatmeten Gase mit einem Druck von etwa 3 bis 20 mbar aus der Leitung an die Umgebung abgeführt' werden, schlägt sich aus der Atemluft Feuchtigkeit nieder, so daß diese Kanäle verstopfen und die Gasableitung unterbunden wird.

Mit einer Vergrößerung des Querschnitts dieser Kanäle kann zwar deren Verstopfen durch niedergeschlagene Feuchtigkeit verhindert werden. Die durch die vergrößerten Kanäle strömende Luft erzeugt jedoch Geräusche, die als lästig empfunden werden. Der aus den vergrößerten Kanälen austretende Luftstrom kann sogar Folgeerkrankungen wie Bindehautentzündungen bewirken.

In der US-A-4 558 708 ist eine Vorrichtung zur Entnahme von trockenen Luftproben, die zu analysieren sind, aus der ausgeatmeten Luft eines Patienten beschrieben. Diese Vorrichtung weist eine Feuchtigkeits-Sperre aus einem feinporigen Werkstoff mit einer Porengröße von 5 µm bis 30 µm auf. Diese Sperre, von der u.a. die Feuchtigkeit in der Atemluft während der Probeentnahme zurückgehalten werden soll, verstopft durch die kondensierende Feuchtigkeit und muß daher häufig ausgewechselt werden.

Aufgabe der Erfindung ist es nun, eine patientenfreundliche und problemlose Ableitung der ausgeatmeten Gase und der Überschußluft bei der künstlichen Beatmung zu schaffen.

Diese Aufgabe wird erfindungsgemäß ausgehend von einer Gaszuführleitung der eingangs beschriebenen Gattung dadurch gelöst, daß der poröse oder feinporige Abschnitt der Gaszuführleitung, über den Gas an die Umgebung abgegeben wird, aus einem hydrophoben Werkstof gefertigt ist oder dessen Poren eine hydrophobe Oberfläche haben.

Das ausgeatmete Gas und die Überschußluft werden über den porös oder feinporig ausgebildeten Abschnitt der Gaszuführleitung an die Umgebung abgegeben, ohne daß ein konzentrierter Luftstom auftritt, da die Luft nach allen Seiten durch die Poren des Materials nach außen strömt. Die dabei entstehenden Geräusche sind in der Regel nicht wahrnehmbar. Es ist auch der Luftstrom außerhalb des porösen Abschnittes, der aus einem Sinterwerkstoff gefertigt und auswechselbar in die Gaszuführleitung eingesetzt sein kann, schon in einem Abstand von wenigen Zentimetern von der Leitung nicht mehr meßbar, wodurch Belästigungen des Patienten bzw. Dritter durch einen konzentrierten Luftstrom vermieden werden. Dabei wird durch die erfindungsgemäße hydrophobe Oberfläche der zur Gasableitung dienenden Poren das Niederschlagen von Feuchtigkeit in denselben und somit deren Verstopfen verhindert, wodurch neben einer patientenfreundlichen zugleich eine störungsfreie Ableitung des ausgeatmeten Gases und der Überschußluft in die Umgebung gewährleistet ist.

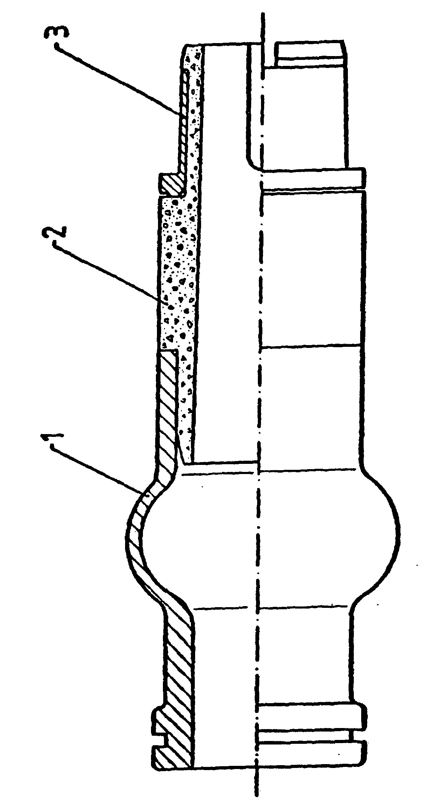

Ein Ausführungsbeispiel der Erfindung wird noch an Hand der Zeichnung beschrieben, in der ein Abschnitt einer Gaszuführleitung zur künstlichen Beatmung eines Patienten in schematischer Teilschnittansicht dargestellt ist.

Der gezeigte Leitungsabschnitt besteht aus einem Gelenkstück 1, das aus einem gummielastischen Material gefertigt und mit dem freien Ende an eine Atemmaske (nicht gezeigt) anschließbar ist. Das andere Ende des Gelenkstückes 1 ist auf einen Abschnitt 2 der Gaszuführleitung gesteckt. Dieser Abschnitt 2 ist aus einem porösen und hydrophoben Werkstoff gefertigt, durch das das ausgeatmete Gas und Überschußluft nach außen in die Umgebung entweichen können. Auf das dem Gelenkstück 1 gegenüberliegende Ende des Abschnittes 2 ist eine drehbar gehaltene Hülse 3 aufgeschnappt, auf der ein mit einem Beatmungsgerät verbundener und nicht dargestellter Schlauch anordenbar ist.

Die Erfindung ist nicht auf die beschriebene und dargestellte Ausführungsform begrenzt. Sie schließt für den Fachmann geläufige Abwandlungen ein. So kann zum Beispiel die Oberfläche der zur Gasableitung vorgesehenen Kanäle hydrophob sein oder der Sinterrohstoff zur Fertigung des porösen Abschnittes der Gaszuführleitung eine hydrophobe Oberfläche aufweisen.

## Patentansprüche

1. Gaszuführleitung, die an eine Atemmaske zur künstlichen Beatmung eines Patienten anschließbar ist und einen Abschnitt aus einem porösen oder feinporigen Werkstoff aufweist, über den Gas an die Umgebung abgegeben wird, **dadurch gekennzeichnet, daß** der Werkstoff hydrophob ist oder dessen Poren eine hydrophobe Oberfläche haben.

2. Gaszuführleitung nach Anspruch 1, **dadurch gekennzeichnet, daß** der poröse oder feinporige Abschnitt (2) auswechselbar in die Gaszuführleitung eingefügt ist.

3. Gaszuführleitung nach Anspruch 1 oder 2, **dadurch gekennzeichnet, daß** der Abschnitt (2) aus einem Sintermaterial gefertigt ist.

## Claims

1. Gas feed conduit which can be connected to a breathing mask for the artificial respiration of a patient and comprises a portion made of a porous or fine pored material via which gas is released to the environment, **characterised in that** the material is hydrophobic or the pores thereof have a hydrophobic surface.

2. Gas feed conduit according to claim 1, **characterised in that** the porous or fine pored portion (2) is replaceably inserted in the gas feed conduit.

3. Gas feed conduit according to claim 1 or 2, **characterised in that** the portion (2) is manufactured from sintered material.

## Revendications

1. Conduite d'amenée de gaz qui peut être raccordée à un masque respiratoire pour la respiration artificielle et qui comporte une section (2) constituée d'une matière poreuse ou d'une matière dotée de minuscules pores par laquelle du gaz est rejeté dans l'environnement, **caractérisée en ce que** ladite matière est hydrophobe ou **en ce que** les pores de ladite matière présente une surface hydrophobe.

2. Conduite d'amenée de gaz selon la revendication 1, **caractérisée en ce que** la section (2) poreuse ou dotée de minuscules pores est insérée de façon amovible dans la conduite d'amenée de gaz.

3. Conduite d'amenée de gaz selon la revendication 1 ou 2, **caractérisée en ce que** la section (2) est fabriquée à partir d'une matière frittée.
